Europäisches Patentamt

European Patent Office

Office européen des brevets

(19)

(11) Publication number: **0 408 277 A2**

# EUROPEAN PATENT APPLICATION

(12)

(21) Application number: **90307459.9**

(22) Date of filing: **09.07.90**

(51) Int. Cl.5: **C07C 263/06**

(30) Priority: **12.07.89 US 378681**

(43) Date of publication of application:
**16.01.91 Bulletin 91/03**

(84) Designated Contracting States:
**AT BE DE FR GB IT NL**

(71) Applicant: **ARCO CHEMICAL TECHNOLOGY INC.**
**3 Christina Centre Suite 902 201 N Walnut Street**
**Wilmington Delaware 19801(US)**

(72) Inventor: **Shawl, Edward T.**
**208 Martroy Lane**
**Wallingford, Pennsylvania 19086(US)**
Inventor: **Zajacek, John G.**
**669 Clovelly Road**
**Devon, Pennsylvania 19333(US)**
Inventor: **Kesling, Haven S., Jr.**
**248 Friendship Road**
**Drexel, Pennsylvania 19026(US)**

(74) Representative: **Cropp, John Anthony David et al**
**MATHYS & SQUIRE 10 Fleet Streeteet**
**London, EC4Y 1AY(GB)**

(54) **Process for the preparation of aliphatic isocyanates.**

(57) A multi-step process for the preparation of an aliphatic mono-, di- or poly- isocyanate from primary aliphatic amines and isocyanic acid to form an aliphatic urea which is then reacted with a dialkyl amine to give a mono-, di- or poly- (dialkyl) urea which is then thermally treated in the presence of a reaction promoter to produce the desired isocyanate.

EP 0 408 277 A2

# PROCESS FOR THE PREPARATION OF ALIPHATIC ISOCYANATES

## FIELD OF THE INVENTION

The present invention relates to a multi-step process for the preparation of aliphatic mono-, di-, and poly- isocyanates from a primary aliphatic amine and isocyanic acid to form an aliphatic urea which urea is reacted with a dialkyl amine to give a mono-, di-, or poly (dialkyl) urea which is then thermally treated in an inert organic solvent in the presence of a reaction promoter to produce the desired aliphatic isocyanate. The aliphatic isocyanates produced by the instant process are of significant industrial importance in the preparation of urethanes or polyurethanes as well as for use or preparation of materials having agricultural applications.

## BACKGROUND OF THE INVENTION

Several aliphatic isocyanates, such as for example, hexamethylene diisocyanate, are prepared commercially by the reaction of an alkyl amine with phosgene to produce the carbamyl chloride derivative which is then thermally decomposed to the desired isocyanate and hydrogen chloride. The use of phosgene raises serious environmental and safety hazards. In addition, its use results in the formation of chloride-containing by-products. A number of processes for the preparation of aliphatic isocyanates not utilizing phosgene have been disclosed which include thermal decomposition such as, for example, the formation and decomposition of 1,6-hexamethylenedicarbamate to the corresponding isocyanate as described in U.S. Patent 4,596,678, June 24, 1986, and the reaction of aliphatic amines with diphenyl carbonate to form an intermediate carbamate derivative which is thermally treated to form the aliphatic isocyanate and phenol as shown in U.S. Patent 3,366,662, January 30, 1968.

The reaction of isocyanic acid with ammonia, primary and secondary amines to prepare aliphatic ureas has been reviewed in an article by D.J. Belson and A.N. Strachan, Chem.Soc. Rev., Vol. II, pp 41-56 (1982).

The formation of trisubstituted ureas has also been disclosed for example, in U.S. Patent No. 2,857,430, October 21, 1958.

Processes have been reported in the literature for the preparation of aromatic mono- and polyisocyanates by the vapor or solvent phase decomposition of substituted ureas using various promoters to help convert the urea groups to isocyanate groups.

The vapor phase production of aromatic isocyanates from symmetrical bis aryl ureas in a solventless system in the presence of hydrogen chloride,phosphorus pentoxide or zinc chloride was described by A. Hofmann in the Proc. Royal Soc., London, Vol.IX p. 274 (1858). By heating a mixture of diphenyl urea with phosphorus pentoxide, zinc chloride or gaseous HCl. Hofmann distilled phenyl isocyanate overhead. No details of the experimental procedure are presented anc yield of isocyanate not given.

A. Hofmann, Chemisch Berichte, Vol.3, pp. 653-658 (1870) described heating diphenyl urea in the presence of phosphoric acid giving yields too small to be considered for the preparation of the isocyanate.

Subsequent work by Iwakura and Nagakubo reported in the Bulletin Tokyo Inst. Technol., Vol. 13, p.25 (1950) and Chemical Abstracts, Vol. 44, p. 3924e (1950) describes the preparation of an aromatic isocyanate (p-ethoxyphenylisocyanate) by heating a solution of bis aryl urea such as bis (p-ethoxyphenyl) urea in the presence of hydrogen chloride gas.

The vapor phase decomposition of bis aryl ureas at 350°C and higher temperatures has been described by W.D. Bennet et al, Journ. Am. Chem. Soc., Vol. 75, p.2101 (1952) and Slocombe et al in U.S. Pat. No. 2,773,086, December 4, 1956 in the presence of gaseous HCl as a promoter. Yields are reported in the 60 to 70% range for the vapor phase reaction and only a 5% yield for liquid phase reaction. A carbamoyl chloride intermediate is formed.

The liquid phase decomposition of trisubstituted ureas to isocyanates has been described by van Landeghem et al, French Patent No. 1,473,821, February 13, 1967; C.J. Hearsey, U. S. Patent No. 3,898,259, August 5, 1975 and Rosenthal et al in the U.S. Patent No. 3,936,484, February 3, 1976. van Landeghem shows thermal decomposition of trisubstituted ureas in an organic solvent having specified dielectric constants at 140° to 170°C with long reaction times of from 6 to 10 hours and modest yields of 60 to 75%. A variety of catalysts are shown but not exemplified or claimed, and include metal salts, such as acetates, stearates, and linoleates of manganese, zinc, cobalt, chromium and vanadium, tertiary amine bases, such as aliphatic, cycloaliphatic, aromatic and mixed tertiary amines, aliphatic heterocyclic amines such as N-methylpiperidine or N, N′-dimethylpiperidine as well as aromatic heterocyclic amines such as pyridine and pyrimidine. Other nitrogen compounds such as imidazole are indicated as being suitable. However, under the reaction conditions described, tertiary amines as shown by van Landeghem do not catalyze urea decomposition.

Rosenthal et al U.S. Patent No. 3,936,484 discloses the thermal decomposition of di- and tri-substituted ureas to isocyanates at temperatures above 230°C in a solvent with short residence times and isocyanate yields of from 60 to 80%.

The Hearsey U.S. Patent No. 3,898,259 describes the introduction of gaseous hydrogen chloride into the liquid phase urea decomposition reaction to give reduced reaction times with isocyanate yields of from 80 - 90%. An excess of gaseous HCl is employed at temperatures of from 100° to 200°C and a by-product carbamoyl chloride intermediate formed.

A.Hentschel et al U.S. Patent No. 4,223,145, September 16, 1980 discloses the formation of an HCl adduct of a trisubstituted urea using at most, a 10% excess of HCl. This adduct is then decomposed in a closed system at from 80 to 180°C.

## SUMMARY OF THE INVENTION

This invention relates to a novel integrated multi-step process for the preparation of aliphatic mono-, di-, or polyisocyanates from aliphatic mono-, di-, or poly- amines and isocyanic acid (HNCO). The aliphatic amines are reacted with isocyanic acid to convert the amino groups to urea groups which aliphatic mono-, di-, or poly- ureas are then reacted with a dialkyl amine, such as diethylamine, to produce a mono-, di-, or polyl (dialkyl) urea product which is slurried in or dissolved in an inert organic solvent and thermally decomposed in the presence of a reaction promoter selected from tertiary amine hydrohalides, organic sulfonic acids and sulfonated aromatic ion exchange resins to produce the corresponding aliphatic isocyanate.

It is an object of the present invention therefore, to provide an improved multi-step process for the preparation of aliphatic mono-, di-, or poly isocyanates from the urea reaction product of an aliphatic amine and isocyanic acid which has been reacted with a dialkyl amine to give a aliphatic mono-, di-, or poly (dialkyl) urea precursor to the corresponding aliphatic isocyanate.

It is another object of this invention to provide an improved reaction (thermal decomposition) system for the conversion of the intermediate mono-, di- or poly (dialkyl) urea to the corresponding aliphatic isocyanate product.

These and other objects and advantages of this invention will become apparent from the description of the invention which follows and from the claims.

## DETAILED DESCRIPTION OF THE INVENTION

In accordance with the present invention an aliphatic mono-, di-, or poly- isocyanate having the formula;

$R(NCO)n$

wherein R is an alkyl radical having from 1 to 20 carbon atoms, which may be linear, branched or cyclic and optionally substituted by aliphatic or aromatic groups or chloride, bromide, alkoxide and nitro groups and n is an integer of from 1 to 4, preferably 1 to 3, is produced by a process which comprises the steps of:

(a) reacting a primary aliphatic mono-, di-, or polyamine having the formula

$R(NH_2)n$

wherein R is described hereinabove and n is an integer of from 1 to 4, preferably 1 to 3, with isocyanic acid at a temperature of from about -30°C to about 200°C preferably from about -10°C to about 150°C in the presence of a solvent or mixture of solvents which are stable and substantially chemically inert to the components of the reaction system, to convert the amino groups to urea groups (-NHCONH_2) to produce an aliphatic mono-, di-, or poly- urea having the structural formula:

$R(NHCONH_2)n$

wherein R and n are as described hereinabove;

(b) reacting said mono-, di-, or poly- urea thus produced with a dialkyl amine having from 1 to 8 carbon atoms in each alkyl group at a temperature of from about 50°C to about 200°C, preferably from about 90°C to about 150°C in the presence of a solvent or mixture of solvents which are also stable and substantially chemically inert to the components of the reaction system to produce a mono-, di-, or poly (dialkyl) urea having the formula:

R(NHCONR'R")n

wherein R and n are as described hereinabove and R' and R" which may be the same or different is each a substituted or unsubstituted monovalent aliphatic radical having independently from 1 to 8 carbon atoms or R' and R" together represent a substituted or unsubstituted divalent aliphatic radical;

(c) thermally decomposing said mono-, di-, or poly(dialkyl) urea dissolved in or slurried in an organic solvent or mixture of solvents, which are stable and substantially chemically inert to the components of the reaction system, in the presence of a reaction promoter selected from the group consisting of a tertiary amine hydrohalide, such as pyridine hydrochloride, organic sulfonic acids and sulfonated aromatic ion exchange resins, at a temperature within the range of from about $50°C$ to about $220°C$, preferably from about $90°C$ to about $150°C$; and

(d) recovering the aliphatic mono-, di-, or polyisocyanate produced.

Representative aliphatic mono-, di- and poly- isooyanates which may be produced by the process of this invention include, for example, the mono- isocyanates containing the methyl, ethyl, propyl, isopropyl, normal, secondary and tertiary butyl, hexyl, 2-methylhexyl, 2-ethylhexyl, decyl, benzyl or cyclohexyl radicals, diisocyanates such as 1,4-butylenediisocyanate, 1,6-hexamethylenediisocyanate, isophoronediisocyanate, xylylidenediisocyanate, cycloaliphatic isocyanates such as cyclohexanediisocyanate, 2,4- and 2,6-hexahydrotoluenediisocyanate, aliphatic-cycloaliphatic diisocyanates such as the isomer mixture of 4,4'-, 2,4'- and 2,2'-dicyclohexylmethane diisocyanate and polyisocyanates derived from the hydrogenated condensation product of aniline with formaldehyde.

Representative primary aliphatic mono-, di- or polyamines which may be reacted with isocyanic acid according to step (a) of the process of the instant invention include, for example, methyl, ethyl, isopropyl, n- and iso- propyl, n-, s-, and tert- butyl and octyl etc amines, benzylamine, 1,6-hexamethylenediamine, cyclohexylamine, and the like.

The isocyanic acid employed in step (a) of the process of the present invention may be produced or generated by known methods such as the pyrolysis of urea or cyanic acid, reaction of cyanate salts such as sodium, potassium or silver cyanate and the like with an acid such as acetic or hydrochloric acid and the like. The isocyanic acid may be generated and used in situ, or it may be distilled away from its source and used in the process of the invention as a gas or dissolved in an appropriate solvent.

In the present process the molar ratio of the ($-NH_2$) groups of the mono-, di-, or poly amines to isocyanic acid is generally one to one. However, an excess of isocyanic acid of up to about 50% may be employed. Alternatively, an excess of up to about 30% ($-NH_2$) groups may be used and any unreacted or partially reacted amine separated from the urea produced and recycled. In the subsequent reaction employing the urea, the molar ratio of the dialkylamine reactant, such as dimethylamine to the urea groups ($-NHCONH_2$) is generally one to one. However, an excess of dialkylamine of from about 10% to a tenfold excess may advantageously be employed to drive the reaction to completion. Unreacted dialkylamine may be easily recovered by distillation for recycle in the reaction.

The dialkylamines or mixtures thereof which may be employed in step (b) of the process of the invention conform to the general formula R'R"HN wherein R' and R" which may be the same or different are alkyl groups having independently from 1 to 8 carbon atoms. Representative dialkylamines include, for example, dimethylamine, diethylamine, methylethylamine, diisopropylamine, dicyclohexylamine, dibutylamine, and the like.

Solvents or mixtures of solvents which are stable and substantially chemically inert to the components of the reaction system are employed in each of the process steps of the present invention. Suitable solvents which may be employed include, for example, the aromatic hydrocarbons such as benzene, toluene, xylene, tetrahydronaphthalene as well as higher alkyl-substituted aromatic hydrocarbons; alkanes and substituted alkanes as well as cycloalkanes having from 5 to 20 carbon atoms such as, for example, n-hexane, n-heptane, octane, nonane, cyclohexane, dodecane, octadecane, 2-methyl- hexane, 2-ethylhexane, methylcyclohexane, cyclopentane and the like; halogenated or nitrated aromatic or aliphatic hydro- carbons such as, for example, methylene chloride, chloroform, carbontetrachloride, 1,2-dichloroethane, chlorobenzene, trichloroethane, tetrachloroethane dichlorobenzene, nitrobenzene dinitrotoluenes and the like; aromatic or aliphatic ether such as for example, diphenylether and dibutylether and the like; tertiary amines such as, for example, pyridine, triethylamine, N-methylpyrolidone and the like. Certain ketones, esters, alcohols as well as water and highly polar solvents, such as sulfolane, dimethysulfoxide, ethylene carbonate or propylene carbonate may also be used. It is not necessary that the amines, the reaction intermediates such as the ureas, dialkylureas or the reaction products be completely miscible with the solvents at the concentrations employed. Advantage may be taken of differing solubilities of the reagents, intermediates and reaction products in the various solvents or mixture of solvents to separate the reaction components or to drive the reaction to increased product. The same solvent or mixture of solvents may be used throughout the reaction system or different solvents or solvent mixtures used in different steps of the process.

The intermediate mono-, di- or poly- urea obtained by the reaction of the amines with isocyanic acid in step (a) may, if desirable, be isolated from the reaction system and further processed according to the process of the invention or it may simply be carried forward as a solution or a slurry without isolation for completion of the process to produce the desired isocyanate. In appropriate solvents, the intermediate mono-, di- or poly urea may be essentially insoluble and the urea easily separated from unreacted amine or partly reacted amine urea by-products by conventional techniques such as filtration, centrifugation, and the like. The separated mono-, di- or poly- urea or the crude intermediate product may be reacted with a dialkyl amine to produce the desired dialkyl urea. The dialkyl amine reactant may be added as a gas, a liquid, a solid or as a solution in solvent. Ammonia generated in this part of the reaction is removed by any convenient means.

The tertiary amine hydrohalide salts employed in the process of the present invention to promote thermal decomposition of the mono-, di- or poly- (dialkyl) ureas to the corresponding isocyanate may be prepared, for example, by reacting the tertiary amine selected with a hydrogen halide such as HCl. Salts of hydrogen fluoride chloride, bromide or iodide may be used. The tertiary amines used to prepare the hydrohalide will conform to the general formula $R,R',R''N$ wherein $R,R'$ and $R''$ are not hydrogen but may be an aliphatic radical having from 1 to 10 carbon atoms, a cycloatiphatic radical such as cyclopentyl, cyclohexyl and cycloheptyl radicals, an aromatic radical, or an aralkyl radical. Such radicals may be substituted with, for example, nitro or halo groups which are non-reactive with the isocyanate produced. Suitable amine salts include, for example, triethylamine hydrochloride, hydrobromide or hydrofluoride, trioctylamine hydro-chloride, hydrobromide or hydrofluoride, N-Methyldiethylamine hydrobromide or hydrochloride, N,N-diethylaniline hydrochloride and N,N-dimethylcyclohexylamine hydrochloride. Hydrohalide salts of heterocyclic tertiary amines and heterocyclic aromatic amines may also be employed. Representative salts include, for example, N-methylpyrrolidine hydrochloride, pyridine hydrochloride or hydrobromide, 3-ethylpyridine hydrochloride, the hydrohalide salt of 1,4- diazabicyclo [2.2.2] octane, 4-chloro- pyridine hydrochloride, $4,4'$-bipyridine dihydrochloride, quinoline hydrochloride or the like. Salts of amine oxides such as 2-chloropyridine N-oxide hydrochloride may also be used as a promoter. In addition, the hydrohalide may be formed with an amine which may be part of a polymer such as polyvinyl pyridine or a resin prepared from tertiary amine groups attached to a styrene divinylbenzene polymer. The tertiary amine hydrohalide is generally employed in the process at a molar ratio of one to one based on the urea groups. However, an excess of the tertiary amine hydrohalide promoter may be used.

The organic sulfonic acid employed in the process of the present invention to promote the thermal decomposition of the mono-, di- or poly- (dialkyl) ureas may be an alkane sulfonic acid or a halogenated alkane sulfonic acid having up to 10 carbon atoms in the alkyl group, or an aromatic sulfonic acid which may contain substituents on the aromatic ring such as halogens, alkyl radicals, aromatic radicals, nitro groups and the like. The organic sulfonic acid may be in the form of an acidic sulfonated aromatic ion exchange resin such as, for example, the sulfonated styrene/divinyl-benzene copolymer (sold, for example, commercially as "Amberlyst 15" by Rohm & Haas Co.) and having a bulk density of approximately 565 g./l. a hydrogen ion concentration of approximately 4.9 milliequivalents/g.dry, a surface area of from about 40 to 50 $m^2$/g. and an average pore diameter of from about 200 to 600 Angstrom units, or an acidic perfluoroalkane sulfonic acid resin such "Nafion" (sold for example, commercially by the DuPont Co.) and having an equivalent weight of between about 110 and 1500, a hydrogen ion concentration of between about 0.7 - 1.0 milliequivalents/g. dry and prepared, for example, by the polymerization of tetrafluoroethylene with a sulfonyl fluoride vinyl ether, followed by saponification with caustic to form the alkali metal salt and treatment with an acid to convert the salt to the sulfonic acid form. Mixtures of the sulfonic acid promoters may be employed but it is preferable to use a single acid promoter to simplify separation and recovery of the aromatic isocyanate produced. Representative organic sulfonic acid promoters suitable for use in the process of this invention include, for example, methane, ethane, butane and hexane sulfonic acids, and the like, trifluoromethane sulfonic acid, benzene sulfonic acid, 1,3-benzene disulfonic acid, p-toluene sulfonic acid, naphthalene sulfonic acid, 4-chloro-3-nitrobenzene sulfonic acid, 3 nitrobenzene sulfonic acid, and the like, as well as the sulfonated aromatic ion exchange resins which include the "Amberlyst 15" and "Nafion" described hereinabove and also include the "Dowex 50" (Dow Chemical), "AG50W" (Bio-Rad), and "Amberlite" (Rohm & Haas) resin materials. The ion exchange resins may be supplied commercially in the hydrogen ion form or the salt form such as the sodium or potassium salt. The salt can readily be converted to the active hydrogen ion form by, for example, treating with aqueous hydrochloric acid, washing with water to a constant pH in the range of 5.5 to 7 and then drying to remove residual water.

The organic sulfonic acid promoter is generally employed in the process of the instant invention at a molar ratio of one to one based on the urea groups to sulfonic acid groups. No advantage is gained by using large excess amounts which may lead to by-product formation.

The process of the present invention can be suitably carried out by adding the mono-, di- or poly-(dialkyl) urea to a solvent or a mixture of solvents comprising the reaction medium. The urea may be soluble in the solvent or solvents or soluble at reaction temperatures or the urea may be in the form of a slurry. Reactions using a sulfonic acid ion exchange resin promoter may also be carried out in a fixed bed of resin by passing a solvent solution of the urea through a bed maintained at the desired reaction temperature. A general procedure for carrying out Step (a) of the process is to charge the primary aliphatic mono-, di- or poly- amine together with a solvent into the reaction vessel. The isocyanic acid is introduced into the reactor as a gas, optionally diluted with an inert gas, as a liquid, or as a solution in an appropriate solvent. Alternatively, the isocyanic acid can be charged to the reactor first together with a solvent and the amine then added as a liquid, a solid, a solution in suitable solvent or as a slurry in a suitable inert liquid. The reaction vessel is heated or cooled as necessary to provide the desired reaction temperature for the appropriate period. Heating and/or cooling means may be employed interior or exterior of the reaction vessel to maintain temperature within desired ranges. The desired reaction product may be recovered by standard filtration and/or distillation procedures.

In the process of the present invention, the step (a) reaction of the amine with isocyanic acid will proceed at temperatures of from about -30°C to about 200°C, preferably from about -10°C to about 150°C. Reaction time is dependent on the temperature but will generally range between about two minutes to several hours. The step (b) reaction of the intermediate mono-, di- or poly- urea and the dialkyl amines will proceed at temperatures of from about 50°C to about 200°C, preferably from about 90°C to about 150°C. The reaction time depends on the temperature but will generally range between about 30 minutes to about 8 hours.

The process of the present invention may be carried out as a batch, semi-continuous or continuous process and the order of addition of the materials and reactants may be varied to suit the particular apparatus reactants and promoter employed.

The reaction of the invention may be carried out in any suitable reactor which is equipped with a means for temperature control and agitation. Heating and/or cooling means may be employed interior or exterior of the reaction vessel to maintain temperature within the desired range.

As indicated hereinabove, the Step (c) thermal decomposition of the mono-, di- or poly- (dialkyl) ureas is carried out at temperatures of from about 50°C to about 220°C, preferably from about 90°C to 150°C. Reaction time is dependent on decomposition temperature but will generally range between about 5 minutes and several hours.

The reaction steps of this invention are generally carried out at atmospheric pressure, but depending on the boiling point of the solvents employed and the isocyanate product, it may be carried out at super-atmospheric or sub-atmospheric pressures. The isocyanate formed may be recovered by filtration, by distillation of either the solvent or the isocyanate, whichever is lower boiling, or by other known methods, and will depend on the solvent employed and the isocyanate produced.

The present invention is more fully illustrated by the following examples, which include particular features of the invention. However, the examples are not to be construed as limiting the invention in any way, it being understood that numerous variations are possible without departing from the spirit and scope of the invention.

EXAMPLE 1

A solution of 11.6g. 0.1 mol, 1,6-hexamethylenediamine in 100g toluene was charged to a 500ml Erlenmeyer flask. The solution was stirred with a magnetic stirrer while 180g of a 5wt% solution of isocyanic acid in toluene containing 9g. (0.21 mol) of isocyanic acid was added quickly at room temperature. Solids precipitated immediately on addition of the isocyanic acid solution. The mixture was stirred for 20 min. at room temperature and then the solids were collected by filtration. The recovered solids, 19.1g, corresponded to a 95% isolated yield of the desired 1,6-hexamethylenebisurea. The identity of the solids was confirmed by comparing the melting point and NMR spectra to a sample of the 1,6-hexamethylenebisurea made by the reaction of 1,6-hexamethylene diisocyanate with ammonia. The solids were then reacted with an excess of diethylamine, 80g, in a pressure vessel for 4 hours at 135°C. Conversion of the 1,6-hexamethylenebis (diethylurea) was confirmed by NMR analysis.

The reaction product was transferred to a 500ml three-neck, round bottom flask equipped with a magnetic stirrer, condenser and thermometer along with 150g of o -xylene. The excess diethylamine was removed from the reaction mixture by distillation and then 20g methane sulfonic acid was added. The mixture was heated to reflux (143°C) for 45 min. with vigorous mixing. The xylene phase was separated from the acid phase and analyzed for 1,6-hexamethylenediisocyanate by infrared analysis. A 90% yield of the diisocyanate was obtained. The o -xylene was stripped on a rotary evaporator to give 11g of the isolated 1,6-hexamethylenediisocyanate.

## EXAMPLE 2

A mixture of 2.9g of 1,6-hexamethylenediamine and 50g pyridine was charged to a 250ml, three-neck, round bottom flask equipped with a mechanical stirrer, condenser, and thermometer and heated to 100°C. 25g of a 10wt% solution of isocyanic acid in 50/50 wt% toluene/pyridine was added quickly to the diamine solution. Solids formed immediately. The mixture was stirred for 15 min. at 100°C and then the entire reaction mixture was transferred to a pressure vessel along with 20g diethylamine. The mixture was heated for 3 hours at 140°C. The excess diethylamine and the solvents were stripped from the product on a rotary evaporator and then 60g toluene and 6g pyridine hydrochloride were added and the mixture was heated for 90 min at 110°C to generate the diisocyanate. The toluene phase, after separation from the salt phase, was analyzed by infrared spectroscopy and showed a 85% yield of the 1,6-hexamethylenediisocyanate.

## EXAMPLE 3

n-Butyl amine, 7.3g (0.1 mol) and 100g chlorobenzene were charged to a 250ml, 3-neck round bottom flask equipped with a magnetic stirrer, condenser, and gas inlet tube. Isocyanic acid, generated by the reaction of sodium cyanate with hydrogen chloride, was distilled away from the sodium cyanate and carried as a gas, diluted with nitrogen, into the round bottom flask which was maintained at -10°C by a refrigerated bath. After a slight excess of isocyanic acid had been added, the reaction was warmed to room temperature and stirred for 30 minutes. The product was transferred to a pressure vessel and 9g dimethylamine was added. The mixture was heated at 120°C for 90 minutes to make N-butyl-N′N′-dimethylurea. The excess dimethylamine was vented from the reactor and the entire reaction mixture was then slurried with 25g of an "Amberlyst" 15 ion exchange resin that had been previously washed first with 3M HCl and then with distilled water and dried in a vacuum oven at 75°C. The reaction mixture was heated at 80°C for 60 minutes and then the mixture was cooled to room temperature and the solid resin was removed by filtration. Analysis of the chlorobenzene solution by infrared analysis showed an overall yield of 75% n-butyl isocyanate based on n-butyl amine.

## EXAMPLE 4

The synthesis of the N-butyl, N′, N′-dimethyl urea was carried out as in Example 3 using 100g o -dichlorobenzene as the solvent. Tne reaction product in o -dichlorobenzene was then slurried with 25g "Nafion NR50" ion exchange resin for 3 hrs at 100°C. The mixture was then cooled to room temperature and the solid resin was removed by filtration. N-Butyl isocyanate, 7.2g, was isolated by distilling it overhead from the o-dichlorobenzene solution.

## EXAMPLES 5 - 9

A number of runs were carried out according to the procedures of Example 1 employing various amines, diamines, or polyamines, dialkylamines, solvents reaction temperatures and cracking promoters. The products were characterized by infrared spectroscopy. The reaction conditions for synthesis of the bis (dialkylurea) compounds are set forth in Table 1 below. These urea compounds were converted to the corresponding isocyanate. The reaction conditions and results for making the corresponding isocyanates from the bis (dialkylurea) compounds of Table 1 are set forth in Table 2 below. Isocyanate yields are based on the starting amine.

TABLE I

| Ex. No. | Amine (g) | Solvent (g) | HNCO(g) Solvent (g) | Temp. (°C) | Time | Dialkyl Amine (g) | Temp. (°C) | Time |
|---|---|---|---|---|---|---|---|---|
| 5 | Xylylenediamine (6.8) | Xylene (100) | 4.5 (as gas) | 100 | 30 min | DBA (50) | 140 | 6 hrs |
| 6 | Hexamethylene Diamine (5.8) | Tetrachloroethane (100)) | 4.5 (as gas) | 140 | 10 min | DMA (25) | 140 | 3 hrs |
| 7 | Hexamethylene diamine (5.8) | Toluene (50) | 4.5 Toluene (20) Pyridine(20) | 30 | 1 hr | DMA (25) | 110 | 8 hrs |
| 8 | HMDA (3.1) | Diphenyl ether (100) | 1.44 | 50 | 30 min | DEA (20) | 130 | 2 hrs |
| 9 | Benzylamine 10.7g | Xylene (50) | 4.5 (as gas) | 30 | 15 min | DEA (50) | 130 | 3 hrs |

HMDA = Hydrogenated methylene diphenylene diamine

DBA = Dibutylamine

DMA = Dimethylamine

DEA = Diethylamine

EP 0 408 277 A2

TABLE 2

| Ex. No. | Promoter (g) | Temp. (°C) | Time | Isocyanate Yield (%) |
|---|---|---|---|---|
| 5 | p-toluene SO$_3$H (20g) | 80 | 2 hrs | 65 |
| 6 | pyridine hydrobromide (16) | 140 | 15 min | 85 |
| 7 | N,N-dimethylaniline hydrobromide (20) | 100 | 30 min | 50 |
| 8 | pyridine hydrochloride (3.5) | 150 | 30 min | 60 |
| 9 | methane sulfonic acid (10) | 90 | 60 min | 83 |

## Claims

1. A process for the preparation of an aliphatic mono-. di- or poly- isocyanate having the general formula:
R(NCO)n
wherein R is an alkyl radical having from 1-20 carbon atoms which may be linear, branched or cyclic and optionally substituted by aliphatic, aromatic, chloride, bromide, alkoxide or nitro groups and n is an integer of from 1 to 4, which comprises the steps of;

(a) reacting a primary aliphatic mono-. di-. or poly- amine having the formula:
R(NH$_2$)n
wherein R is as described hereinabove and n is an integer of from 1 to 4, with isocyanic acid at a temperature of from about -30°C to about 200°C in the presence of an inert organic solvent or mixture of solvents to convert the amino groups to urea groups and produce an aliphatic mono-, di-, or poly- urea having the structural formula:
R(NHCONH$_2$)n

(b) reacting said aliphatic mono-, di-, or poly- urea with a dialkylamine having from 1 to 8 carbon atoms in each alkyl group at a temperature of from about 50°C to about 200°C in the presence of an inert solvent or mixture of solvents to produce a mono-, di-, or poly (dialkyl) urea having the formula:
R(NCHONR$'$R$''$)n
wherein R and n are as hereinabove describe and R$'$ and R$''$ which may be the same or different is each a substituted or unsubstituted aliphatic radical having independently from 1 to 8 carbon atoms or R$'$ and R$''$ may together represent a substituted or unsubstituted divalent aliphatic radical

(c) thermally decomposing said mono-, di-, or poly (dialkyl) urea dissolved in or slurried in an organic solvent or mixture of solvents which are stable and substantially chemically inert to the components of the reaction system at a temperature of from about 50°C to about 220°C in the presence of a reaction promoter selected from tertiary amine hydrohalides, organic sulfonic acids and sulfonated aromatic ion exchange resins; and

(d) recovering the aliphatic mono-, di- or poly isocyanate.

2. A process according to Claim 1 wherein the temperature of reacting the amine with isocyanic acid in step (a) is in the range of from about -10°C to about 150°C.

3. A process according to Claim 1 or Claim 2 wherein the temperature of reacting the mono-, di- or poly- urea with a dialkyl amine in step (b) is in the range of from about 90°C to about 150°C

4. A process according to any one of Claims 1 to 3 wherein the aliphatic mono-, di- or poly- amine is selected from hexamethylenediamine, n-butyl amine, benzylamine, xylenediamine and hydrogenated methylene diphenylene diamine.

5. A process according to any one of Claims 1 to 4 wherein the dialkyl amine reactant of step (b) is selected from dimethylamine, dibutylamine and diethylamine.

6. A process according to any one of Claims 1 to 5 wherein the temperature of thermally decomposing the mono-, di- or poly- (dialkyl) urea is in the range of from about 90°C to 150°C.

7. A process according to any one of Claims 1 to 6 wherein the organic solvents employed in the steps of the process are selected from toluene, pyridine, xylenes, o-dichlorobenzene, chlorobenzene, tetrachloroethane and diphenyl ether.

8. A process according to any one of Claims 1 to 7 wherein the tertiary amine hydrohalide promoter is selected from pyridine hydrochloride, pyridine hydrobromide and N,N-dimethylaniline hydrobromide.

9. A process according to any one of Claims 1 to 7 wherein the organic sulfonic acid is selected from methane sulfonic acid and p-toluene sulfonic acid.

10. A process according to any one of Claims 1 to 7 wherein the sulfonated aromatic ion exchange resin is selected from sulfonated styrene/divinylbenzene copolymer resins and acidic perfluoroalkane sulfonic acid resins.

11. A process for the preparation of 1,6-hexamethylene diisocyanate which comprises the steps of:

(a) reacting 1,6-hexamethylenediamine with isocyanic acid at a temperature of from about -10°C to about 150°C in the presence of an inert organic solvent to convert the amine to 1,6-hexamethylenebis urea;

(b) reacting said 1,6-hexamethylenebis urea with diethylamine at a temperature of from about 90°C to about 150°C to produce 1,6-hexamethylenebis (diethylurea);

(c) thermally decomposing said 1,6-hexamethylenebis (diethylurea) in an inert organic solvent in the presence of a reaction promoter selected from methane sulfonic acid, pyridine hydrochloride and pyridine hydrobromide at a temperature of from about 90°C to about 150°C and;
(d) recovering the 1,6-hexamethylene diisocyanate.